(19) Europäisches Patentamt
European Patent Office
Office européen des brevets

(11) Numéro de publication: **0 353 172 A2**

(12) **DEMANDE DE BREVET EUROPEEN**

(21) Numéro de dépôt: **89420284.5**

(22) Date de dépôt: **27.07.89**

(51) Int. Cl.$^5$: **C 08 K 3/16**
C 08 K 3/24, C 08 K 5/00,
C 08 K 5/09, C 08 L 67/04,
C 02 F 1/76

(30) Priorité: **29.07.88 FR 8810533**

(43) Date de publication de la demande:
**31.01.90 Bulletin 90/05**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Jorda, Rafael**
**30, rue Claude Jusseaud**
**F-69110 Sainte-Foy-Les-Lyon (FR)**

**Porte, Hugues**
**6, Chemin de Crépieux**
**F-69300 Caluire (FR)**

**Torres, Ghislaine**
**104, rue Ney**
**F-69006 Lyon (FR)**

(74) Mandataire: **Seugnet, Jean Louis et al**
**RHONE-POULENC CHIMIE Service Brevets Chimie**
**Centre de Recherches des Carrières B.P. 62**
**F-69192 Saint-Fons Cédex (FR)**

(54) **Composition polymère érodable contenant de l'iode.**

(57) La présente invention concerne une composition polymère érodable comportant un composé d'iode organique et/ou minéral, solide à température ambiante, non toxique, soluble dans l'eau et n'inhibant pas le catalyseur de durcissement.

Le polymère hydrolysable est choisi parmi un polylactique, un polyglycolique et un copolymère lactique/glycolique de poids moléculaire compris entre 10 000 et 800 000, ou leurs mélanges.

Les compositions sont utilisables pour le traitement des eaux avec libération continue et contrôlée de l'iode pour le traitement et l'éradication des maladies dues à une carence en iode.

EP 0 353 172 A2

**Description**

## COMPOSITION POLYMERE ERODABLE CONTENANT DE L' IODE

La présente invention concerne une composition polymère érodable contenant de l'iode ainsi qu'un procédé de traitement des eaux d'usage domestique et de boissons à l'aide de cette composition.

On estime actuellement à plusieurs centaines de millions le nombre de sujets présentant une déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc .....).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'àlors sont demeurées inéfficaces.

Ces actions visent essentiellement d'une part :

- l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu' il n'est pas parfaitement emballé.

et d'autre part :

- l'injection intra-musculaire d'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induite par l'injection d'une dose nécessairement supra-physiologique.

On a par ailleurs décrit dans le brevet belge BE-A-889 680 l'introduction d'oligo-éléments, dont l'iode, dans l'eau de boisson des ruminants sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A2 347 567, US-A-2 743 208 et US-A-3 408 295.

Il existe également de très nombreux brevets décrivant l'utilisation de systèmes polymères en particulier de silicone pour la libération contrôlée d'un principe actif au moyen par exemple d'un système transdermique, (brevet américain US-A-4 053 580) ou par absorption buccale notamment pour des ruminants (brevet français FR-A-2 560 768).

On peut également citer GB-A-1 437 835 qui enseigne l'encapsulation d'un composé d'iode dans du PVC, du polyamide, du polyéthylène et du polypropylène et US-A-2 743 208 qui décrit un système matriciel flottant sur l'eau et constitué d'un composé d'iode enrobé d'urée et de polyoxyde d'éthylène.

Enfin par le brevet américain US-A-4 384 960 il est décrit la mise en pastilles d'iode $I_2$ dans une bouteille en plastique dans laquelle l'eau pénètre par une membrane. L'eau solubilise l'iode. Le rôle de la membrane est seulement d'éviter que l'iode en pastilles ne sorte pas de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car $I_2$ est un inhibiteur bien connu des catalyseurs de durcissement des élastomères silicones vulcanisables à température ambiante (voir en particulier la publication de W.D. MORAIN et al. Plastic and Reconstructive Surgery 59, 2, 215-222 (1977).

En outre dans le système du brevet US-A-4 384 960 il n'y a pas de contrôle de la libération de l'iode d'une part, et, d'autre part, l'iodation de l'eau se fait par addition discontinue ou en continu de quelques gouttes d'eau très iodée (à saturation) contenue dans la bouteille, dans un récipient quelconque contenant de l'eau non traitée. Il est clair que la solution proposée par US-A-4 384 960 est imparfaite par le fait notamment qu'il s'agit d'une technique individuelle qui, comme l'injection intra-musculaire d'iode, nécessite une éducation et une mobilisation massive des populations.

La présente invention a précisément pour but de proposer une composition polymère érodable contenant de l'iode utilisable pour le traitement continu des eaux d'usage domestique, en particulier dans les systèmes d'adduction, de traitement des eaux dans les puits et les forages et qui permette de relarguer (libérer) une quantité contrôlée et adaptée d'iode en vue d'assurer l'éradication des diverses maladies dues à une carence en iode.

Elle a également pour but de proposer une composition polymère érodable contenant de l'iode qui immergée de façon appropriée dans les endroits contenant l'eau à traiter, en particulier les puits et forages, relargue (libère) de façon continue, de préférence pendant quelques mois, une quantité appropriée d'iode sous une forme et à une dose thérapeutiquement active et efficace pour soigner les diverses maladies dues à une carence en iode.

Elle a également pour but de proposer une composition polymère érodable contenant de l'iode qui,

immergée de façon appropriée dans les endroits contenant l'eau à traiter n'ait aucune action secondaire indésirable et néfaste sur le plan chimique et biologique sur les eaux à traiter.

Elle a également pour but de proposer une composition polymère érodable contenant de l'iode présentant une forme adaptée au milieu dans lequel se trouve l'eau à traiter, cette forme étant adaptée en particulier aux puits et/ou forages et présentant un système d'introduction dans les puits et/ou forages permettant son remplacement aisé.

Elle a également pour but de proposer une composition polymère érodable contenant de l'iode apporté par un composé de l'iode qui soit compatible avec la matrice polymère érodable.

Ces buts et d'autres sont atteints par la présente invention qui concerne en effet une composition polymère érodable, caractérisée en ce qu'elle comporte :

(A) : un polymère hydrolysable choisi parmi un polylactique, un polyglycolique et un copolymère lactique/glycolique de poids moléculaire compris entre 10 000 et 800 000, ou leurs mélanges,

(B) : au moins un composé d'iode organique et/ou minéral sous une forme solide à température ambiante, soluble dans l'eau, non toxique,

On peut introduire de 5 à 130 parties, de préférence de 10 à 100 parties de composé d'iode (B) pour 100 parties de polymère (A).

Dans ce qui suit ou ce qui précède, sauf mentions contraires, les pourcentages et parties sont en poids.

Comme composé minéral de l'iode on peut utiliser :

- les iodures ou iodates de formules générales :

$(M^{a+})\ (I^-)_a$

et $(M^{a+})\ (IO_3^-)_a$

dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NZ_4)^+$, dont les radicaux Z identiques ou différents représentent un radical alkyle linéaire ou ramifié en $C_1$-$C_{20}$ ou un atome d'hydrogène, tel que l'ion ammonium $NH^+_4$.

Les cations $M^{a+}$ et $NZ_4^+$, sont choisis de telle sorte que l'iodure ou l'iodate correspondant soit un solide ou un liquide à température ambiante, soit soluble dans l'eau et soit non toxique.

Comme iodures et iodates on peut en particulier utiliser ceux de formules :

$Na\ I$ , $Na\ IO_3$,

$K\ I$ , $K\ IO_3$,

$Mg\ I_2$ , $Mg\ I_2,\ 8H_2O$,

$Mg(IO_3)_2,\ 4H_2O$,

$NH_4\ I$

$Fe\ I_2,\ 4H_2O$

$Mn\ I_2$

Ces sels peuvent contenir de l'eau d'hydratation ou de l'eau de constitution.

Comme composé de l'iode à la fois organique et minéral on peut par exemple utiliser l'iodobehenate de calcium de formule :

$(C_{21}\ H_{42}\ ICO_2)Ca$

Comme composé organique de l'iode on peut citer la polyvinylpyrrolidone iodée.

Pour des raisons de facilité de mise en oeuvre les composés d'iode NaI et $KIO_3$ sont les plus préférés.

Tous les composés de l'iode tels que définis ci-dessus, lorsqu' ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 100 µg/l à température ambiante.

En particulier dans les pays en développement, l'eau à usage domestique (boisson, lavage, irrigation, etc .....) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite à l'aide d'une pompe à corps immergé.

Cette eau se renouvelle principalement en fonction de l'utilisation du forage ou de la saison. En effet en saison des pluies le forage est traditionnellement moins utilisé. Par contre en saison sèche, le forage débite environ 12 heures par jour, soit une quantité comprise entre 5 et 10 $m^3$ par jour pendant environ six mois.

En règle générale, un puits peut être asséché deux fois durant la journée au moment de la saison sèche ce qui correspond avec ces données statistiques moyennes, à un maximum d'utilisation de 5 à 10 $m^3$ .

De nombreuses études montrent que dans les zones de grande endémie goîtreuse, le taux pré-existant d'équivalent en iode dans l'eau des forages ou des puits est inférieur à 2 microgrammes par litre (2 µg/l). Il est estimé actuellement qu'un apport journalier d'environ 100 µg d'équivalent iode par jour et par personne serait

suffisant pour prévenir le développement d'un goître endémique et sans doute environ 150 µg lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 µg, de préférence environ 100 µg, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 µg/l d'iode, ce qui correspond à 50 µg d'équivalent iode par litre et par personne, ce qui nécessite que la composition de silicone relargue 720 mg/j d'équivalent d'iode, soit 270 g d'équivalent iode à relarguer pendant une année.

Dans ce qui suit ou ce qui précède, sauf indications contraires, les parties et pourcentages sont en poids.

De façon surprenante et inattendue on a en effet découvert selon la présente invention qu'il est possible d'ajouter dans une composition polymère érodable des quantités importantes de composé d'iode sous une forme solide ou liquide tel que défini ci-dessus, à savoir de 5 à 130 parties, de préférence de 10 à 100 pour 100 parties de polymère hydrolysable choisi parmi un polymère polylactique, un polymère polyglycolique et un copolymère lactique/glycolique ou leurs divers mélanges et d'obtenir ainsi un produit qui présente des caractéristiques mécaniques suffisantes pour l'application envisagée et qui, immergé dans l'eau permette d'assurer un relargage continu et contrôlé d'iode de préférence pendant quelques mois par érosion continue de la composition provoquée par hydrolyse lente du polymère (A).

De façon à maîtriser la libération du principe actif, il est avantageux de présenter la composition de polymère érodable sous la forme de modules (éléments) élémentaires de formes variées tels que des cubes, des parallélépipèdes rectangles, des cylindres, des sphères dont les paramètres fondamentaux sont les suivants :

- la nature du composé d'iode,
- la nature et le poids moléculaire du polymère utilisé,
- le diamètre moyen (granulométrie) g des particules du composé d'iode dans le cas préféré où ce dernier est un solide,
- la teneur t de composé d'iode au sein de la matrice,
- le rapport R de la surface au volume du module.

La nature du composé d'iode et sa granulométrie définissent la vitesse de diffusion (r) à travers la matrice, du principe actif.

Plus g est petit et plus v est lent et inversement.

Plus t est grand et plus le flux de principe actif est élevé et inversement.

Plus R est grand et plus le flux élevé de principe actif est grand et inversement.

Plus le poids moléculaire du polymère est faible, plus l'hydrolyse du polymère est rapide et plus le flux de principe actif libéré est élevé.

Plus le polymère est amorphe, plus l'hydrolyse du polymère est rapide et plus le flux du principe actif libéré est élevé.

L'homme de métier, par des expériences de routine, peut sans difficulté aboutir rapidement au résultat visé en extrapolant le temps d'élution théorique qui correspondra au temps réel de diffusion du principe actif.

Pour NaI et $KIO_3$ qui sont les composés d'iode préférés, g, t et R peuvent être avantageusement choisis dans les zones suivantes :

- g compris entre 1 et 300 µm,
- t compris entre 10 et 100 parties en poids de composé d'iode pour 100 parties de (A),
- R compris entre 0,5 et 50 pour une forme cylindrique.

Il est en outre souhaitable que le composé d'iode soit dispersé de façon homogène au sein de la matrice polymère.

Le polymère (A) utilisable dans le cadre de la présente invention est choisi parmi les polymères polylactiques, polyglycoliques et les copolymères lactique/glycolique de poids moléculaire compris entre 10 000 et 800 000.

Plus spécifiquement le polymère (A) répond à la formule générale :

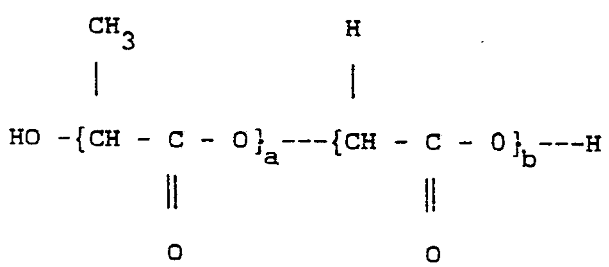

dans laquelle a est un nombre entier compris entre 0 et 10 000 inclus,
b est un nombre entier compris entre 0 et 10 000 inclus,

a + b est supérieur à 150 et inférieur à 10 000.

Les valeurs indiquées ci-dessus pour a et b correspondent à des poids moléculaires inclus dans la fourchette 10 000-800 000.

Les polymères (A) présentent la propriété d'être hydrolysables. En présence d'eau et du composé (B) le polymère (A) se dégrade lentement et confère à la composition sa propriété d'érodabilité qui permet de libérer lentement le composé d'iode dans l'eau à traiter.

Les polymères (A) sont des produits pour la plupart disponibles dans le commerce et leurs procédés de préparation décrits dans de nombreux brevets parmi lesquels on peut citer : US-A-2 676 945, US-A-4 273 920, FR-A-1 425 333, FR-A-2 086 047 et EP-A-171 907.

Les procédés d'obtention du polymère sont principalement de deux types par condensation ou par polymérisation.

Selon le procédé de polycondensation on place l'acide lactique, l'acide glycolique ou un mélange des deux monomères dans un réacteur fermé.

On effectue la polycondensation de préférence sans catalyseur en augmentant la température et en diminuant la pression simultanément. Le temps de réaction peut aller par exemple de 5 à 120 heures ; on peut faire passer la température de 20 à 220 °C et abaisser simultanément la pression de la pression atmosphérique à 0,02 KPa ou moins.

Selon le procédé de polymérisation on utilise des dimères cycliques des acides lactiques ou glycoliques nommés lactides ou glycolides ou leurs mélanges.

Le lactide utilisé peut être du lactide L(-) optiquement pur ou du lactide DL racémique.

Ceci conditionne la stéréorégularité du polylactide obtenu. Ainsi dans le cas du lactide L(-) on obtient un polymère semi-cristallin, alors qu'avec du lactide DL, on obtient un polymère amorphe.

On effectue la polymérisation en présence d'un catalyseur.

Les conditions de durée de réaction, de température et de pression sont les suivantes :

- temps de réaction compris entre 5 et 120 heures,
- température comprise entre 110 et 150 °C et
- pression inférieure à 0,02 KPa.

Les caractéristiques de poids moléculaire, de polydispersité et de cristallinité du polymère obtenu selon l'un ou l'autre procédé, sont contrôlées par les conditions expérimentales et la composition des monomères de départ.

Les compositions selon l'invention peuvent être extrudées ou moulées suivant des formes variées, par exemple sous forme de modules (éléments) unitaires.

Les modules (éléments) de formes variées peuvent être tenus immergés dans les eaux à traiter suivant une quantité telle que la composition assure une libération continue et contrôlée d'iode, de préférence pendant quelques mois. Au bout de cette période les modules (éléments) sont remplacés.

L'exemple suivant illustre l'invention sans en limiter la portée.

- EXEMPLE :

SYNTHESE DU POLYLACTIQUE UTILISE :

On charge dans un ballon de 250 ml 105 g de DL-Lactide recristallisé deux fois dans l'acétate d'éthyle. On prépare une solution d'octoate d'étain $Sn-(O-\underset{\underset{O}{\|}}{C}-C_7-H_{15})_2$ dans l'hexane de concentration C = 0,073 mole/litre.

On verse 5 ml de la solution d'octoate d'étain dans le ballon réactionnel puis on évapore l'hexane sous vide.

On purge 3 fois le ballon à l'azote sec en alternant le vide, azote puis on établit un vide de 13 Pa dans le ballon. Celui-ci est scellé et placé dans une étuve à 140 °C pendant 100 heures.

Après refroidissement, le polymère formé est dissout dans le dichlorouréthane, précipité dans le méthanol, récupéré et séché sous vide à 60 °C pendant 24 heures.

Le polymère obtenu est un polylactique amorphe PLA-DL de poids moléculaire 100 000.

On chauffe 100 g de ce polylactique ainsi préparé sous agitation et atmosphère d'azote. Lorsque la température atteint 230 °C, on ajoute 25 g de NaI de granulométrie comprise entre 100 et 200 microns.

On obtient un mélange homogène que l'on coule dans des moules en acier recouverts d'huile silicone (agent anti-adhérant) et de forme cylindrique (diamètre égal à 24 mm). Le cylindre obtenu à base de polylactique/NaI est refroidi rapidement et démoulé.

Protocole expérimental :

La composition polymère contenant le NaI est découpée à la longueur désirée (26 mm) et immergée dans un récipient contenant 1 000 ml d'eau distillée, thermostatée à 20 °C.

Le récipient est équipé d'un système d'agitation magnétique, animé d'un mouvement de rotation lent (100 tr/mn) assurant l'homogénéité de la solution. Il est fermé de façon à minimiser l'évaporation de l'eau. Des prélèvements journaliers de 5 ml sont réalisés dans les premiers temps de l'élution puis ceux-ci sont espacés et deviennent hebdomadaires.

La concentration en iodure, libérée par jour, est déterminée par un dosage avec une électrode spécifique à iodure. On immerge l'électrode dans la solution à doser et on lit le potentiel électrochimique de la solution. Une

courbe d'étalonnage préalablement établie avec des solutions de iodure de concentration connue, permet de calculer la concentration (C en mg/l) en iodure de la solution dosée.

Caractéristiques du cylindre immergé :

| | |
|---|---|
| Diamètre | = 24 mm |
| Hauteur | = 26 mm |
| Masse | = 14,5 g |
| Quantité de NaI | = 2,9 g |

Dans le tableau ci-dessous sont rassemblés les résultats de la cinétique d'élution.

| TEMPS | Q : QUANTITE CUMULEE DE NaI LIBERE (g) | 100 x Q/Qo (%) |
|---|---|---|
| 1 | 0,046 | 1,6 |
| 2 | 0,151 | 5,2 |
| 3 | 0,580 | 20 |
| 6 | 0,963 | 33,2 |
| 8 | 1,160 | 40 |
| 10 | 1,407 | 48,5 |
| 16 | 1,630 | 56,2 |
| 23 | 1,937 | 66,8 |
| 35 | 2,477 | 85,4 |
| 42 | 2,789 | 96 |

Q cumulé correspond à la quantité de NaI éluée à l'instant t sous forme ionique $Na^+$, $I^-$. Le temps d'élution total du système présenté ci-dessus est de l'ordre de 50 jours.

**Revendications**

1.- Composition polymère érodable, caractérisée en ce qu'elle comporte :
(A) : au moins un polymère hydrolysable choisi parmi un polylactique, un polyglycolique ou leurs mélanges et un copolymère lactique/glycolique de poids moléculaire compris entre 10 000 et 800 000,
(B) : un composé d'iode organique et/ou minéral, sous forme solide à température ambiante, soluble dans l'eau, non toxique,
et en ce qu'elle comporte de 5 à 130 parties en poids de composé de l'iode (B) pour 100 parties en poids de polymère (A).

2.- Composition polymère selon la revendication 1, caractérisée en ce que le composé de l'iode (B) est un iodure ou iodate de formules générales :
$(M^{a+}) (I^-)_a$
et $(M^{a+}) (IO^-_3)_a$
dans lesquelles a est un nombre entier supérieur ou égal à 1 et M est un cation choisi parmi un métal alcalin, un métal alcalino-terreux, un métal de transition et un ammonium quaternaire $(NZ_4)^+$ dont les radicaux Z identiques ou différents représentent un radical alkyle linéaire ou ramifié en $C_1$-$C_{20}$ ou un atome d'hydrogène.

3.- Composition selon la revendication 1, caractérisée en ce que le composé de l'iode (B) est choisi parmi :
$Na I$, $Na IO_3$,
$K I$, $K IO_3$,
$Mg I_2$, $Mg I_2, 8H_2O$,
$Mg(IO_3)_2, 4H_2O$,
$NH_4 I$
$Fe I_2, 4H_2O$
$Mn I_2$

4.- Composition selon la revendication 1, caractérisée en ce que le composé de l'iode (B) est l'iodobehenate de calcium.

5.- Composition selon la revendication 1, caractérisée en ce que le composé de l'iode (B) est la polyvinylpyrrolidone iodée.

6. - Composition selon l'une quelconque des revendications précédentes, caractérisée en ce que le polymère (A) répond à la formule générale :

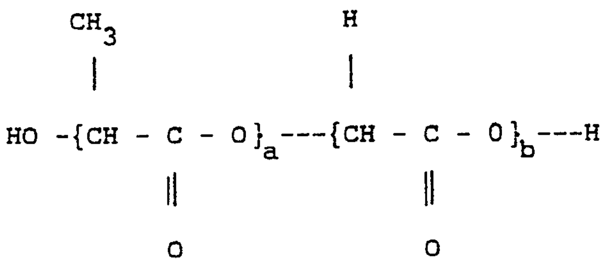

dans laquelle a est un nombre entier compris entre 0 et 10 000 inclus,
b est un nombre entier compris entre 0 et 10 000 inclus,
a + b est supérieur à 150 et inférieur à 10 000.

7. - Procédé de traitement d'eau, caractérisé en ce qu'on immerge une quantité adaptée d'une composition polymère telle que définie à l'une quelconque des revendications 1 à 6 en vue de libérer dans l'eau une quantité continue et contrôlée d'iode.